Europäisches Patentamt

European Patent Office

Office européen des brevets

(11) Publication number: 0 329 981
A1

# EUROPEAN PATENT APPLICATION

(21) Application number: 89101592.7

(22) Date of filing: 31.01.89

(51) Int. Cl.4: A61F 2/16

(30) Priority: 11.02.88 IT 1938188

(43) Date of publication of application:
30.08.89 Bulletin 89/35

(84) Designated Contracting States:
AT BE CH DE ES FR GB GR IT LI LU NL SE

(71) Applicant: ADVANCE MEDICAL S.r.l.
Via Giannone 9
I-20154 Milano(IT)

(72) Inventor: Liffredo, Renato
Via Giannone 9
I-20154 Milano(IT)

(74) Representative: Modiano, Guido et al
MODIANO, JOSIF, PISANTY & STAUB
Modiano & Associati Via Meravigli, 16
I-20123 Milan(IT)

(54) Intraocular lens with chromatic and absorption-diagram correction.

(57) An intraocular lens (1) with chromatic and absorption-diagram correction, comprises: at least one inner body (2) of optically transparent material, having a substantially lenticular shape, and an outer body (3), also of optically transparent material, which substantially completely includes the inner body and also has a substantially lenticular shape. The inner body and the outer body are constituted of materials with different refraction and chromatic-dispersion indexes to substantially eliminate chromatic aberration for at least two wavelengths and spherical aberration for at least two corresponding points.

Fig.3

EP 0 329 981 A1

## INTRAOCULAR LENS WITH CHROMATIC AND ABSORPTION-DIAGRAM CORRECTION

The present invention relates to an intraocular lens with chromatic and absorption-diagram correction.

As is known, intraocular lenses are constituted by a substantially lenticular body of optionally transparent material having a small diameter, usually comprised between 5 and 7 mm, which is implanted in the eye after cataract surgery and restores normal vision since it performs the optical function of the natural crystalline lens which has been removed.

Intraocular lenses generally have fixing elements, such as protrusion or the like, also of transparent material, which engage inside the eye so as to keep the intraocular lens correctly arranged on the optical axis of the eye. Said fixing elements have different configurations according to the intended position of the intraocular lens, i.e. they differ from one another depending on whether the lens is to be placed in front of the pupil or behind it.

The lenticular body can be biconvex, planoconvex or concavo-convex, with a power in aqueous humor usually comprised between 10 and 30 diopters, so as to converge light rays of an image, to focus said image substantially on the eye's retina.

Although known intraocular lenses effectively restore vision in cataract surgery subjects, preventing the use of very powerful lenses, they have some disadvantages.

Known intraocular lenses in fact have considerable chromatic aberration and an absorption spectrum for ultraviolet and visible radiation which differs considerably from that of the natural crystalline lens.

The radiation absorption spectrum could be modified by appropriately coloring the intraocular lens, but the substances used for said coloring are very often biologically incompatible with the eye and can give rise to sensitization and to rejection phenomena.

Another problem occurring in the use of intraocular lenses is the phenomenon of blinding. In vision with dilated pupils, for example under poor lighting, the light rays which affect the peripheral portions of the lenticular body are in fact refracted and reflected erratically, multiplying bright point-shaped images and thus causing a bothersome disturbance to normal vision.

The aim of the present invention is to solve the above described problems by providing an intraocular lens which reduces chromatic aberration and has an absorption diagram similar to that of the natural crystalline lens.

Within the scope of this aim, an object of the invention is to provide an intraocular lens which eliminates blinding.

Another object of the invention is to provide an intraocular lens which offers adequate assurances against sensitization and rejection on the part of the human body.

This aim, as well as these and other objects which will become apparent hereinafter, are achieved by an intraocular lens with chromatic and absorption-diagram correction, characterized in that it comprises: at least one inner body, of optically transparent material, having a substantially lenticular shape, and an outer body of optically transparent material which substantially completely includes said inner body and also has a substantially lenticular shape, said inner body and said outer body being constituted of materials with different indexes of refraction and chromatic dispersion adapted to substantially eliminate chromatic aberration for at least two wavelengths and spherical aberration for at least two corresponding points.

Further characteristics and advantages of the invention will become apparent from the description of some preferred but not exclusive embodiments of the intraocular lens according to the invention, illustrated only by way of non-limitative example in the accompanying drawings, wherein:

figures 1 and 2 are schematic views of the production step of an intraocular lens according to a first aspect of the invention;

figure 3 is a sectional view of the intraocular lens, taken along a diametrical plane;

figure 4 is a front view of the intraocular lens of figure 3;

figures 5 to 7 are sectional views of other embodiments of the intraocular lens according to the invention, taken along a diametrical plane.

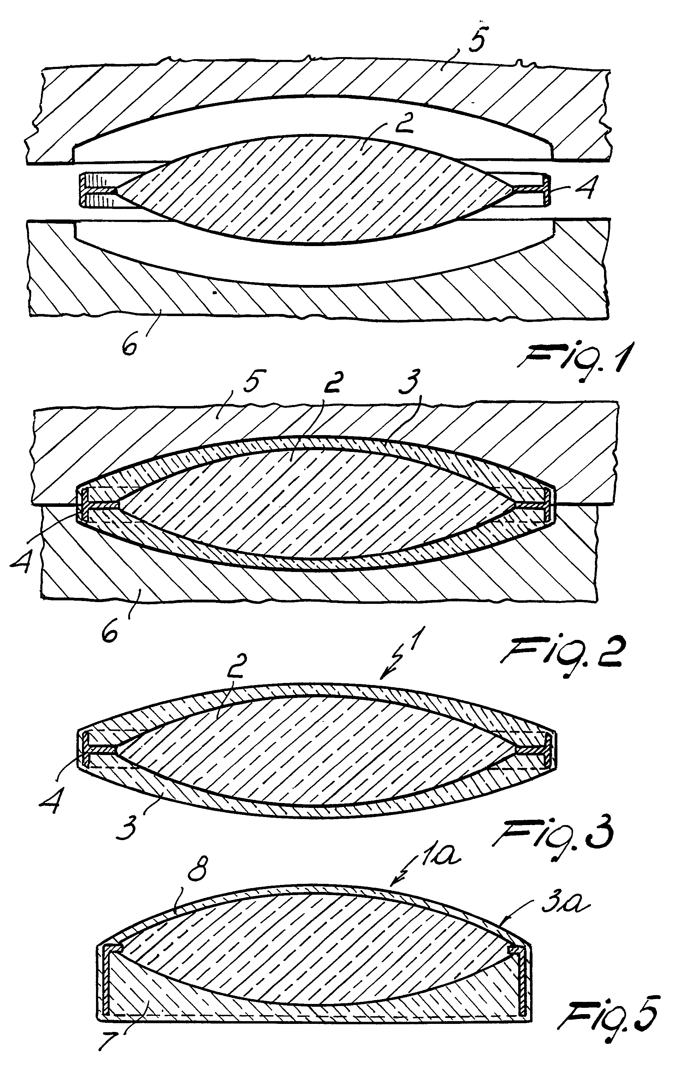

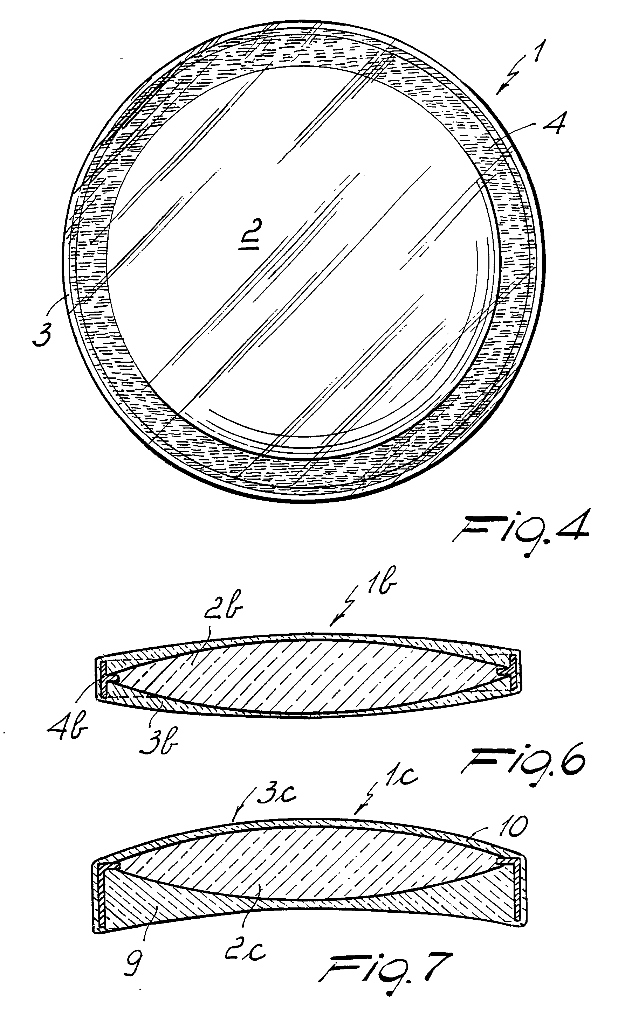

With particular reference to figure 3, the intraocular lens according to the invention, generally indicated by the reference numeral 1, comprises at least one inner body 2 made in a known manner of optically transparent material and having a substantially lenticular shape; the inner body 2 is totally included inside an outer body 3, also of optically transparent material and having a lenticular shape. The materials constituting the outer body and the inner body are chosen so that the two bodies have mutually different refraction and chromatic-dispersion indexes and, by appropriately choosing their radii of curvature, chromatic aberration can be eliminated for two wavelengths and spherical aberration can be eliminated for two corresponding points, for example the point at infinity on the optical axis of the lens in the object field and the focal point in the image field, though the outer body and the inner body have spherical-profile surfaces.

The radii of curvature and the refraction and chromatic-dispersion indexes, which are preferably different for the two bodies which compose the lens, are determined and chosen in a known manner according to the wavelengths for which aberrations are to be eliminated.

Advantageously, in order to give the intraocular lens an absorption spectrum similar to that of the natural crystalline lens, the inner body 2 is colored, for example, with known chromophores so as to absorb radiations of certain wavelengths which must be eliminated or attenuated. It is usually necessary to eliminate ultraviolet radiation completely, eliminate most of the blue radiation and attenuate all of the remaining part of the visible spectrum.

The chromophores may be concentrated in a layer of uniform thickness or be uniformly dispersed in the inner body. In the first case the obtained absorption is proportional to the intensity of the incident radiation as the aperture of the pupil varies, while in the second case the percentage of absorption is higher for a small pupil aperture and decreases as the diameter of the pupil rises, automatically compensating the absorption according to the intensity of the incident radiation.

Advantageously, in order to eliminate the phenomenon of blinding, an optically opaque annular body 4 is arranged around the inner body 2 and it, too, is totally included inside the outer body 3. In this manner the phenomenon of erratic refraction of the rays incident to the intraocular lens proximate to its edge is avoided when viewing under poor lighting, i.e. with dilated pupils, since the rays which affect said portion of the lens are completely absorbed or reflected.

By virtue of the fact that both the inner body 2 and the annular body 4 are included inside the outer body 3, the materials and chromophores used to produce the inner body and the annular body can adequately comply with the optical requirements, since they need not necessarily to comply with biocompatibility requirements, which are required only for the outer body.

The inner body 2 and the annular body 4 are produced in a known manner, and the outer body 3 is produced, as illustrated in particular in figures 1 and 2, by correctly arranging the bodies 2 and 4 in an appropriate mold with two half-shells 5 and 6 and by introducing in the mold the synthetic material which constitutes the outer body in the liquid or semi-liquid state. Said material is polymerized in a known manner, enclosing the inner body 2 and the annular body 4.

As illustrated in figure 3, the outer body can be biconvex and its thickness can increase from the center of the lens towards its edge, constituting a diverging lens on each side of the inner body.

Figure 5 illustrates a second embodiment of the intraocular lens according to the invention, indicated by the reference numeral 1a, wherein the outer body 3a comprises a portion in the shape of a plano-concave lens 7 arranged on one side of the inner body 2a. On the opposite side of the inner body 2a the outer body has a protective layer 8 so as to nonetheless include the inner body 2a.

Figure 6 illustrates a third embodiment of the intraocular lens, indicated by the reference numeral 1b, wherein the annular body 4b extends partially inside the inner body 2b. In this embodiment the inner body 2b and the outer body 3b have different radii of curvature with respect to the lens according to the first embodiment.

Figure 7 illustrates a fourth embodiment of the intraocular lens, indicated by the reference numeral 1c, wherein the outer body 3c has a biconcave-lens portion 9 on one side of the inner body 2c. On the opposite side, similarly to the lens of figure 5, the outer body 3c is constituted by a protective layer 10 so as to completely include the inner body 2c.

The intraocular lens according to the invention can have, in a known manner, coupling protrusion or expansions possibly provided monolithically with the outer body to anchor it inside the eye. Said known protrusion or expansions have not been illustrated for the sake of simplicity.

In practice it has been observed that the intraocular lens according to the invention fully achieves the intended aim, since it provides chromatic and absorption-diagram corrections which are very similar to the natural crystalline lens.

Another advantage is that the most optically suitable materials can be used for the inner body and for the possible anti-blinding annular body, with no problems of biocompatibility.

The intraocular lens thus conceived is susceptible to numerous modifications and variations, all of which are within the scope of the inventive concept; all the details may furthermore be replaced with technically equivalent elements.

In practice the materials employed, as well as the dimensions, may be any according to the requirements and to the state of the art.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Intraocular lens with chromatic and absorption-diagram correction, characterized in that it comprises at least one inner body (2, 2b, 2c) of optically transparent material, having a substantially lenticular shape, and an outer body (3, 3a, 3b, 3c) of optically transparent material, substantially completely including said inner body and also having a substantially lenticular shape, said inner body and said outer body being constituted of materials with different refraction and chromatic-dispersion indexes adapted to substantially eliminate chromatic aberration for at least two wavelengths and spherical aberration for at least two corresponding points.

2. Intraocular lens, according to claim 1, characterized in that said outer body has different outer radii of curvature with respect to the radii of curvature of said inner body.

3. Intraocular lens, according to claims 1 and 2, characterized in that said inner body and said outer body are in the shape of a biconvex lens.

4. Intraocular lens, according to claim 3, characterized in that the thickness of said outer body increases from the center towards its edges.

5. Intraocular lens, according to claims 1 and 2, characterized in that said inner body is in the shape of a biconvex lens and in that said outer body has a portion in the shape of a biconcave lens.

6. Intraocular lens, according to one or more of the preceding claims, characterized in that an optically opaque annular body (4, 4b) is arranged around said inner body in said outer body.

7. Intraocular lens, according to one or more of the preceding claims, characterized in that said inner body is made of transparent material with the addition of chromophores.

8. Intraocular lens, according to one or more of the preceding claims, characterized in that said chromophores are uniformly dispersed inside said inner body.

9. Intraocular lens, according to one or more of the preceding claims, characterized in that said chromophores are concentrated in a layer of uniform thickness inside said inner body.

10. Intraocular lens, according to one or more of the preceiding claims, characterized in that said outer body and said inner body have a spherical curvature.

11. Intraocular lens, according to one or more of the preceding claims, characterized in that said outer body is made of synthetic material polymerized on said inner body.

Fig.1

Fig.2

Fig.3

Fig.5

_Fig.4_

_Fig.6_

_Fig.7_

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-3 428 895 (SCHMIDT) <br> * claims 1,3-6; figures 2-4 * | 1 | A 61 F 2/16 |
| A | | 2,3 | |
| A | FR-A-2 601 872 (CESKOSLOVENSKA AKADEMIE VED) <br> * figure 4; claim 4 * | 1 | |
| A | EP-A-0 212 616 (KINGSTON TECHN., INC.) <br> * claims 1,10; figures 2,3 * | 1 | |
| A | US-A-4 198 714 (JENSEN) <br> * column 5, lines 26-32; figure 4 * | 1 | |
| A | US-A-4 172 297 (SCHLEGEL) <br> * figure 3; column 6, lines 36-46 * | 1,6 | |

TECHNICAL FIELDS
SEARCHED (Int. Cl.4)

A 61 F 2/00

The present search report has been drawn up for all claims

| Place of search | Date of completion of the search | Examiner |
|---|---|---|
| BERLIN | 12-05-1989 | KANAL P K |